# EUROPÄISCHE PATENTANMELDUNG

(11) **EP 1 103 611 A1**
(43) Veröffentlichungstag der Anmeldung: **30.05.2001**
(21) Anmeldenummer: 00125527.2
(22) Anmeldetag: 22.11.2000
(51) Int. Cl.: C12N 15/52, C12N 9/00, C12P 13/04, C12P 13/14

(54) **Für die Gene sucC und sucD kodierende Nukleotidsequenzen**

(30) Priorität: 25.11.1999 DE 19956686
(71) Anmelder: Degussa AG, 40474 Düsseldorf (DE)
(72) Erfinder: Möckel, Bettina, Dr., 40597 Düsseldorf (DE); Pfefferle, Walter, Dr., 33790 Halle (Westf.) (DE); Marx, Achim, Dr., 33613 Bielefeld (DE)

(57) **Zusammenfassung**

Die Erfindung betrifft für die Gene sucC und sucD kodierende Polynukleotide, die Polynukleotidsequenzen enthalten, ausgewählt aus der Gruppe
a) Polynukleotid, das mindestens zu 70 % identisch ist mit einem Polynukleotid, das für ein Polypeptid kodiert, das die Aminosäuresequenz von SEQ ID No. 2 enthält,
b) Polynukleotid, das mindestens zu 70 % identisch ist mit einem Polynukleotid, das für ein Polypeptid kodiert, das die Aminosäuresequenz von SEQ ID No. 3 enthält,
c) Polynukleotid, das für ein Polypeptid kodiert, das eine Aminosäuresequenz enthält, die zu mindestens 70% identisch ist mit der Aminosäuresequenz von SEQ ID No. 2,
d) Polynukleotid, das für ein Polypeptid kodiert, das eine Aminosäuresequenz enthält, die zu mindestens 70% identisch ist mit der Aminosäuresequenz von SEQ ID No. 3,
e) Polynukleotid, das komplementär ist zu den Polynukleotiden von a), b), c) oder d), und
e) Polynukleotid, enthaltend mindestens 15 aufeinanderfolgende Nukleotide der Polynukleotidsequenz von a), b), c), d) oder e),
ein Verfahren zur fermentativen Herstellung von L-Aminosäuren unter Verwendung von coryneformen Bakterien, in denen die Gene abgeschwächt vorliegen und die Verwendung der Polynukleotidsequenzen als Hybridisierungssonden.

## Beschreibung

Gegenstand der Erfindung sind für die Gene sucC und sucD kodierenden Nukleotidsequenzen aus coryneformen Bakterien und ein Verfahren zur fermentativen Herstellung von Aminosäuren, insbesondere L-Lysin und L-Glutamat, unter Verwendung von Bakterien, in denen das sucC- und/oder sucD-Gen abgeschwächt wird.

### Stand der Technik

L-Aminosäuren, insbesondere L-Lysin und L-Glutamat, finden in der Humanmedizin und in der pharmazeutischen Industrie, in der Lebensmittelindustrie und ganz besonders in der Tierernährung Anwendung.

Es ist bekannt, daß Aminosäuren durch Fermentation von Stämmen coryneformer Bakterien, insbesondere Corynebacterium glutamicum (C. glutamicum), hergestellt werden. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren.

Seit einigen Jahren werden ebenfalls Methoden der rekombinanten DNA-Technik zur Stammverbesserung von L-Aminosäure produzierenden Stämmen von Corynebacterium eingesetzt.

### Aufgabe der Erfindung

Die Erfinder haben sich zur Aufgabe gestellt, neue Maßnahmen zur verbesserten fermentativen Herstellung von Aminosäuren, insbesondere L-Lysin und L-Glutamat, bereitzustellen.

### Beschreibung der Erfindung

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan und L-Arginin gemeint. Besonders bevorzugt sind L-Lysin und L-Glutamat.

Gegenstand der Erfindung ist ein isoliertes Polynukleotid, enthaltend eine Polynukleotidsequenz, ausgewählt aus der Gruppe
a) Polynukleotid, das mindestens zu 70 % identisch ist mit einem Polynukleotid, das für ein Polypeptid kodiert, das die Aminosäuresequenz von SEQ ID No. 2 enthält,
b) Polynukleotid, das mindestens zu 70 % identisch ist mit einem Polynukleotid, das für ein Polypeptid kodiert, das die Aminosäuresequenz von SEQ ID No. 3 enthält,
c) Polynukleotid, das für ein Polypeptid kodiert, das eine Aminosäuresequenz enthält, die zu mindestens 70% identisch ist mit der Aminosäuresequenz von SEQ ID No. 2,
d) Polynukleotid, das für ein Polypeptid kodiert, das eine Aminosäuresequenz enthält, die zu mindestens 70% identisch ist mit der Aminosäuresequenz von SEQ ID No. 3,
e) Polynukleotid, das komplementär ist zu den Polynukleotiden von a), b), c) oder d), und
f) Polynukleotid, enthaltend mindestens 15 aufeinanderfolgende Nukleotide der Polynukleotidsequenz von a), b), c), d) oder e),
wobei das Polypeptid bevorzugt die Aktivität der Succinyl-CoA Synthetase aufweist.

Gegenstand der Erfindung ist ebenfalls das Polynukleotid gemäß Anspruch 1, wobei es sich bevorzugt um eine replizierbare DNA handelt, enthaltend:
(i) die Nukleotidsequenz, gezeigt in SEQ ID No. 1, oder
(ii) mindestens eine Sequenz, die der Sequenz (i) innerhalb des Bereichs der Degeneration des genetischen Kodes entspricht, oder
(iii) mindestens eine Sequenz, die mit der zur Sequenz (i) oder (ii) komplementären Sequenz hybridisiert, und gegebenenfalls
(iv) funktionsneutralen Sinnmutationen in (i).

Weitere Gegenstände sind
ein Polynukleotid gemäß Anspruch 4, enthaltend die Nukleotidsequenz wie in SEQ ID No. 1 dargestellt,
ein Polynukleotid gemäß Anspruch 1, wobei das Polynukleotid eine in coryneformen Bakterien replizierbare, bevorzugt rekombinante DNA ist,
ein Vektor, enthaltend Teile des erfindungsgemäßen Polynukleotids, mindestens aber 15 aufeinanderfolgende Nukleotide der beanspruchten Sequenz,
und coryneforme Bakterien, in denen das sucC- und/oder sucD-Gen, insbesondere durch eine Insertion oder Deletion, abgeschwächt ist.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige sucC- und/oder sucD-Gen mit der Polynukleotidsequenz entsprechend SEQ ID No. 1 enthalten mit einer Sonde, die die Sequenz des genannten Polynukleotids gemäß SEQ ID No. 1 oder ein Fragment davon enthält und Isolierung der genannten DNA-Sequenz.

Polynukleotide, die die Sequenzen gemäß der Erfindung enthalten, sind als Hybridisierungs-Sonden für RNA, cDNA und DNA, um cDNA geeignet, um Nukleinsäuren beziehungsweise Polynukleotide oder Gene in voller Länge zu isolieren, die für Succinyl-CoA Synthetase kodieren und solche cDNA oder Gene zu isolieren, die eine hohe Ähnlichkeit der Sequenz mit der der Succinyl-CoA Synthetase Gene aufweisen.

Polynukleotide, die die Sequenzen gemäß der Erfindung enthalten, sind weiterhin als Primer geeignet, mit deren Hilfe mit der Polymerase Kettenreaktion (PCR) DNA von Genen hergestellt werden kann, die für Succinyl-CoA Synthetase kodieren.

Solche als Sonden oder Primer dienende Oligonukleotide enthalten mindestens 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Nukleotiden.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäuren enthalten.

Die Polypeptide gemäß Erfindung schließen die Polypeptide gemäß SEQ ID No. 2 und SEQ ID No. 3, insbesondere solche mit der biologischen Aktivität der Succinyl-CoA Synthetase und auch solche ein, die zu wenigstens 70% identisch sind mit dem Polypeptid gemäß SEQ ID No. 2 oder SEQ ID No. 3, bevorzugt zu wenigstens 80% und besonders zu wenigstens 90 % bis 95 % Identität mit dem Polypeptid gemäß SEQ ID No. 2 oder SEQ ID No. 3 und die genannte Aktivität aufweisen.

Die Erfindung betrifft weiterhin ein Verfahren zur fermentativen Herstellung von Aminosäuren, ausgewählt aus der Gruppe L-Asparagin, L-Threonin, L-Serin, L-Glutamat, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan und L-Arginin, insbesondere L-Lysin und L-Glutamat, unter Verwendung von coryneformen Bakterien, die insbesondere bereits die Aminosäuren insbesondere L-Lysin und/oder L-Glutamat produzieren und in denen die für das sucC- und/oder sucD-Gen kodierenden Nukleotidsequenzen abgeschwächt, insbesondere auf niedrigem Niveau exprimiert werden.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Die Mikroorganismen, die Gegenstand der vorliegenden Erfindung sind, können Aminosäuren, insbesondere L-Lysin, aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind besonders die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme, wie beispielsweise die L-Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM 5714.

Die neuen, für das Enzym Succinyl-CoA Synthetase (EC 6.2.1.5) kodierenden Gene sucC und sucD von C. glutamicum wurden isoliert.

Zur Isolierung des sucC- und/oder des sucD-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde.

Börmann et al. (Molecular Microbiology 6(3), 317-326 (1992)) wiederum beschreiben eine Genbank von C. glutamicum ATCC13032 unter Verwendung des Cosmids pHC79 (Hohn und Collins, Gene 11, 291-298 (1980)). O'Donohue (The Cloning and Molecular Analysis of Four Common Aromatic Amino Acid Biosynthetic Genes from Corynebacterium glutamicum. Ph.D. Thesis, National University of Ireland, Galway, 1997) beschreibt die Klonierung von C. glutamicum Genen unter Verwendung des von Short et al. (Nucleic Acids Research, 16: 7583) beschriebenen λ Zap Expressionssystems.

Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind wie beispielsweise der Stamm DH5a (Jeffrey H. Miller: "A Short Course in Bacterial Genetics, A Laboratory Manual and Handbook for Escherichia coli and Related Bacteria", Cold Spring Harbour Laboratory Press, 1992).

Die mit Hilfe von Cosmiden oder anderen λ-Vektoren klonierten langen DNA-Fragmente können anschließend wiederum in gängige, für die DNA-Sequenzierung geeignete Vektoren subkloniert werden.

Methoden zur DNA-Sequenzierung sind unter anderem bei Sanger et al. (Proceedings of the National Academy of Sciences of the United States of America, 74:5463-5467, 1977) beschrieben.

Die erhaltenen DNA-Sequenzen können dann mit bekannten Algorithmen bzw. Sequenzanalyse-Programmen wie z.B. dem von Staden (Nucleic Acids Research 14, 217-232(1986)), dem GCG-Programm von Butler (Methods of Biochemical Analysis 39, 74-97 (1998)) dem FASTA-Algorithmus von Pearson und Lipman (Proceedings of the National Academy of Sciences USA 85,2444-2448 (1988)) oder dem BLAST-Algorithmus von Altschul et al. (Nature Genetics 6, 119-129 (1994)) untersucht und mit den in öffentlich zugänglichen Datenbanken vorhandenen Sequenzeinträgen verglichen werden. Öffentlich zugängliche Datenbanken für Nukleotidsequenzen sind beispielsweise die der European Molecular Biologies Laboratories (EMBL, Heidelberg, Deutschland) oder die des National Center for Biotechnology Information (NCBI, Bethesda, MD, USA).

Die neuen für das sucC- und sucD-Gen kodierende DNA-Sequenzen von C. glutamicum wurden gefunden, die als SEQ ID No. 1 Bestandteil der vorliegenden Erfindung ist. Weiterhin wurde aus den vorliegenden DNA-Sequenzen mit den oben beschriebenen Methoden die Aminosäuresequenz der entsprechenden Proteine abgeleitet. In SEQ ID No. 2 und SEQ ID No. 3 sind die sich ergebenden Aminosäuresequenzen des sucC- und des sucD Genproduktes dargestellt.

Kodierende DNA-Sequenzen, die sich aus SEQ ID No. 1 durch die Degeneriertheit des genetischen Codes ergeben, sind ebenfalls Bestandteil der Erfindung. In gleicher Weise sind DNA-Sequenzen, die mit SEQ ID No. 1 oder Teilen von SEQ ID No. 1 hybridisieren, Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID No. 1 ergeben.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflußt bzw. bestimmt wird. Die Hybridisierungsreaktion wird vorzugsweise bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein 5x SSC-Puffer bei einer Temperatur von ca. 50 - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50 - 68°C eingestellt wird. Es ist gegebenenfalls möglich die Salzkonzentration bis auf 0,1x SSC zu senken. Durch schrittweise Erhöhung der Hybridisierungstemperatur in Schritten von ca. 1 - 2°C von 50 auf 68°C können Polynukleotidfragmente isoliert werden, die beispielsweise mindestens 70% oder mindestens 80% oder mindestens 90% bis 95% Identität zur Sequenz der eingesetzten Sonde besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Anleitungen zur Amplifikation von DNA-Sequenzen mit Hilfe der Polymerase-Kettenreaktion (PCR) findet der Fachmann unter anderem im Handbuch von Gait: Oligonukleotide synthesis: A Practical Approach (IRL Press, Oxford, UK, 1984) und bei Newton und Graham: PCR (Spektrum Akademischer Verlag, Heidelberg, Deutschland, 1994).

Es wurde gefunden, daß coryneforme Bakterien nach Abschwächung des sucC- und/oder sucD-Gens in verbesserter Weise L-Aminosäuren, insbesondere L-Lysin, produzieren.

Zur Erzielung einer Abschwächung können entweder die Expression des suc- und/oder sucD-Gens oder die katalytischen Eigenschaften der Enzymproteine herabgesetzt oder ausgeschaltet werden. Gegebenenfalls können beide Maßnahmen kombiniert werden.

Die Verringerung der Genexpression kann durch geeignete Kulturführung oder durch genetische Veränderung (Mutation) der Signalstrukturen der Genexpression erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z. B. in der Patentanmeldung WO 96/15246, bei Boyd und Murphy (Journal of Bacteriology 170: 5949 (1988)), bei Voskuil und Chambliss (Nucleic Acids Research 26: 3548 (1998), bei Jensen und Hammer (Biotechnology and Bioengineering 58: 191 (1998)), bei Patek et al. (Microbiology 142: 1297 (1996)) und in bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung bzw. Herabsetzung der katalytischen Eigenschaften von Enzymproteinen führen, sind aus dem Stand der Technik bekannt; als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ("Die Threonindehydratase aus Corynebacterium glutamicum: Aufhebung der allosterischen Regulation und Struktur des Enzyms", Berichte des Forschungszentrums Jülichs,Jül-2906, ISSN09442952, Jülich, Deutschland, 1994) genannt. Zusammenfassende Darstellungen können bekannten Lehrbüchern der Genetik und Molekularbiologie wie z. B. dem von Hagemann ("Allgemeine Genetik", Gustav Fischer Verlag, Stuttgart, 1986) entnommen werden.

Als Mutationen kommen Transitionen, Transversionen, Insertionen und Deletionen in Betracht. In Abhängigkeit von der Wirkung des Aminosäureaustausches auf die Enzymaktivität wird von Fehlsinnmutationen ("missense mutations") oder Nichtsinnmutationen ("nonsense mutations") gesprochen. Insertionen oder Deletionen von mindestens einem Basenpaar in einem Gen führen zu Rasterverschiebungsmutationen ("frame shift mutations"), in deren Folge falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie wie z. B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Eine gebräuchliche Methode, Gene von C. glutamicum zu mutieren, ist die von Schwarzer und Pühler (Bio/Technology 9, 84-87 (1991)) beschriebene Methode der Gen-Unterbrechung ("gene disruption") und des Gen-Austauschs ("gene replacement").

Bei der Methode der Gen-Unterbrechung wird ein zentraler Teil der Kodierregion des interessierenden Gens in einen Plasmidvektor kloniert, der in einem Wirt (typischerweise E. coli), nicht aber in C. glutamicum replizieren kann. Als Vektoren kommen beispielsweise pSUP301 (Simon et al., Bio/Technology 1, 784-791 (1983)), pK18mob oder pK19mob (Schäfer et al., Gene 145, 69-73 (1994)), pK18mobsacB oder pK19mobsacB (Jäger et al., Journal of Bacteriology 174: 5462-65 (1992)), pGEM-T (Promega Corporation, Madison, WI, USA), pCR2.1-TOPO (Shuman (1994). Journal of Biological Chemistry 269:32678-84; US-Patent 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173:4510-4516) in Frage. Der Plasmidvektor, der das zentrale Teil der Kodierregion des Gens enthält, wird anschließend durch Konjugation oder Transformation in den gewünschten Stamm von C. glutamicum überführt.

Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross-over"-Ereignisses wird die Kodierregion des betreffenden Gens durch die Vektorsequenz unterbrochen und man erhält zwei unvollständige Allele, denen jeweils das 3'- bzw. das 5'-Ende fehlt. Diese Methode wurde beispielsweise von Fitzpatrick et al. (Applied Microbiology and Biotechnology 42, 575-580 (1994)) zur Ausschaltung des recA-Gens von C. glutamicum verwendet. Das sucC- und/oder sucD-Gen können auf diese Weise ausgeschaltet werden.

Bei der Methode des Genaustaussches ("gene replacement") wird eine Mutation wie z.B. eine Deletion, Insertion oder Basenaustausch in dem interessierenden Gen in-vitro hergestellt. Das hergestellte Allel wird wiederum in einen für C. glutamicum nicht replikativen Vektor kloniert und dieser anschließend durch Transformation oder Konjugation in den gewünschten Wirt von C. glutamicum überführt. Nach homologer Rekombination mittels eines ersten, Integration bewirkenden "cross-over"-Ereignisses und eines geeigneten zweiten, eine Excision bewirkenden "cross-over"-Ereignisses im Zielgen bzw. in der Zielsequenz erreicht man den Einbau der Mutation bzw. des Allels. Diese Methode wurde beispielsweise von Peters-Wendisch (Microbiology 144, 915 - 927 (1998)) verwendet, um das pyc-Gen von C. glutamicum durch eine Deletion auszuschalten. In das sucC- und/oder sucD-Gen kann auf diese Weise eine Deletion, Insertion oder ein Basenaustausch eingebaut werden.

In das sucC- und/oder sucD -Gen kann auf diese Weise eine Deletion, Insertion oder ein Basenaustausch eingebaut werden.

Zusätzlich kann es für die Produktion von L-Aminosäuren, insbesondere L-Lysin, vorteilhaft sein, zusätzlich zur Abschwächung des sucC- und/oder sucD-Gens eines oder mehrere Enzyme des jeweiligen Biosyntheseweges, der Glykolyse, der Anaplerotik, des Zitronensäure-Zyklus oder des Aminosäure-Exports zu verstärken, insbesondere zu überexprimieren.

So kann für die Herstellung von L- Lysin und/oder L-Glutamat neben der Abschwächung des sucC- und/oder sucD-Gens gleichzeitig eines oder mehrere der Gene, ausgewählt aus der Gruppe
- das für die Dihydrodipicolinat-Synthase kodierende dapA-Gen (EP-B 0 197 335),
- das für die Glyceraldehyd-3-Phosphat Dehydrogenase kodierende gap-Gen (Eikmanns (1992), Journal of Bacteriology 174:6076-6086),
- das für die Triosephosphat Isomerase kodierende Gen tpi (Eikmanns (1992), Journal of Bacteriology 174:6076-6086),
- das für die 3-Phosphoglycerat Kinase kodierende Gen pgk (Eikmanns (1992), Journal of Bacteriology 174:6076-6086),
- das für die Pyruvat Carboxylase kodierende pyc-Gen(Eikmanns (1992), Journal of Bacteriology 174:6076-6086),
- das für die Malat:Chinon Oxidoreduktase kodierende mqo-Gen (Molenaar et al., European Journal of Biochemistry 254, 395-403 (1998)),
- das für eine feed-back resistente Aspartatkinase kodierende Gen lysC (Accession No.P26512),
- das für den L-Lysin-Export kodierende lysE-Gen (DE-A-195 48 222),
- das für das Zwal-Protein kodierende Gen zwal (DE: 19959328.0, DSM 13115)
verstärkt, insbesondere überexprimiert werden.

Weiterhin kann es für die Produktion von L-Lysin und/oder - L-Glutamat vorteilhaft sein, neben der Abschwächung des sucC- und/oder sucD -Gens gleichzeitig eines oder mehrere der Gene, ausgewählt aus der Gruppe
- das für die Phosphoenolpyruvat-Carboxykinase kodierende Gen pck (DE 199 50 409.1, DSM 13047),
- das für die Glucose-6-Phosphat Isomerase kodierende Gen pgi (US 09/396,478, DSM 12969),
- das für die Pyruvat-Oxidase kodierende Gen poxB (DE:1995 1975.7, DSM 13114),
- das für das Zwa2-Protein kodierende Gen zwa2 (DE: 19959327.2, DSM 13113)
- abzuschwächen, insbesondere die Expression zu verringern.

Weiterhin kann es für die Produktion von Aminosäuren, insbesondere L-Lysin und/oder L-Glutamat, vorteilhaft sein, neben der Abschwächung des sucC- und/oder sucD-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die das Polynukleotid gemäß Anspruch 1 enthaltenden Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren, insbesondere L-Lysin, kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnußöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden.

Als Stickstoffquelle können organische Stickstoff- haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch "reversed phase" HPLC erfolgen, so wie bei Lindroth et al. (Analytical Chemistry (1979) 51: 1167-1174) beschrieben.

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences, USA 84:2160-2164), bezogen von der Firma Stratagene (La Jolla, USA, Produktbeschreibung SuperCos1 Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Res. 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A laboratory Manual, Cold Spring Harbor) beschrieben durchgeführt, wobei die Zellen auf LB-Agar (Lennox, 1955, Virology, 1:190) mit 100 µg/ml Ampicillin ausplattiert wurden. Nach Inkubation über Nacht bei 37°C wurden rekombinante Einzelklone selektioniert.

### Beispiel 2

### Isolierung und Sequenzierung der Gene sucC und sucD

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 µg/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academies of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems(Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZero1-Derivate wurden zu einem zusammenhängenden Contig assembliert. Die computergestützte Kodierbereichsanalyse wurden mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID No. 1 dargestellt. Die Analyse der Nukleotidsequenz ergab ein offenes Leseraster von 1206 Basenpaaren, welches als sucC-Gen bezeichnet wurde, sowie ein offenes Leseraster von 882 Basenparen, welches als sucD bezeichnet wurde. Das sucC-Gen kodiert für ein Polypeptid von 402 Aminosäuren, welches in SEQ ID No. 2 dargestellt ist. Das sucD-Gen kodiert für ein Polypeptid von 294 Aminosäuren, welches in SEQ ID No. 3 dargestellt ist.

### Beispiel 3

### 3.1 Herstellung eines Integrationsvektors für die Integrationsmutagenese des sucC-Gens

Aus dem Stamm ATCC 13032 wurde nach der Methode von Eikmanns et al. (Microbiology 140: 1817 - 1828 (1994)) chromosomale DNA isoliert. Aufgrund der aus Beispiel 2 für C. glutamicum bekannten Sequenz des sucC-Gens wurden die folgenden Oligonukleotide für die Polymerase Kettenreaktion ausgewählt:

sucC-in1:
5`CGC GCG AAT CGT TCG TAT 3`
sucC-in2:
5`CGC CAC CAA TGT CTA GGA 3`

Die dargestellten Primer wurden von der Firma MWG Biotech (Ebersberg, Deutschland) synthetisiert und nach der Standard-PCR-Methode von Innis et al. (PCR Protocols. A Guide to Methods and Applications, 1990, Academic Press) mit der Pwo-Polymerase der Firma Boehringer Mannheim (Deutschland, Produktbeschreibung Pwo DNA Polymerase, Product No. 1 644 947) die PCR Reaktion durchgeführt. Mit Hilfe der Polymerase-Kettenreaktion ermöglichen die Primer die Amplifikation eines ca. 0,55 kb großen internen Fragmentes des sucC-Gens. Das so amplifizierte Produkt wurde in einem 0,8%igen Agarosegel elektrophoretisch geprüft.

Das amplifizierte DNA Fragment wurde mit dem Zero Blunt™ Kit der Firma Invitrogen Corporation(Carlsbad, CA, USA; Katalog Nummer K2700-20) in den Vektor pCR®Blunt II (Bernard et al., Journal of Molecular Biology, 234: 534-541 (1993)) ligiert.

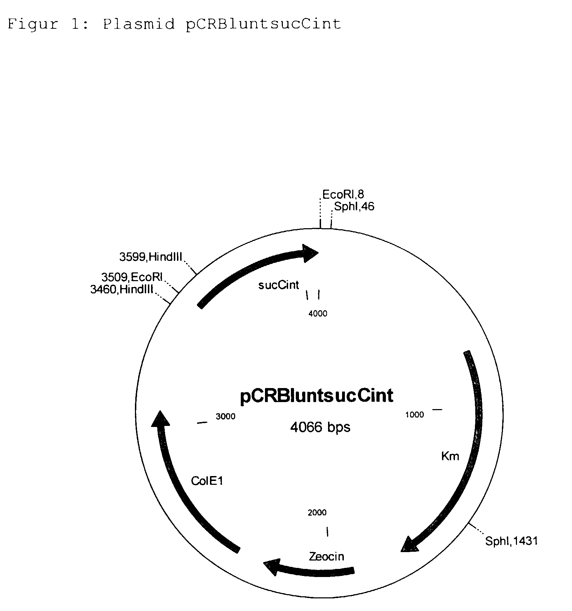

Anschließend wurde der E. coli Stamm TOP10 mit dem Ligationsansatz (Hanahan, In: DNA Cloning. A Practical Approach, Vol.I, IRL-Press, Oxford, Washington DC, USA, 1985) elektroporiert. Die Selektion von Plasmid-tragenden Zellen erfolgte durch Ausplattieren des Transformationsansatzes auf LB Agar (Sambrook et al., Molecular Cloning: A Laboratory Manual, 2^{nd} Ed., Cold Spring Harbor Laboratory Press, Cold Spring Harbor, N.Y., 1989), der mit 25 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und anschließender Agarosegel-Elektrophorese (0,8%) überprüft. Das Plasmid wurde pCRBluntsucCint genannt und ist in Figur 1 dargestellt.

### 3.2 Deletion des sucD-Gen

Hierzu wurde aus dem Stamm ATCC13032 nach der Methode von Tauch et al. (1995, Plasmid 33:168-179) chromosomale DNA isoliert. Aufgrund der aus Beispiel 2 für C.glutamicum bekannten Sequenz des sucD-Gens wurden die im folgenden beschriebenen Oligonukleotide für die Erzeugung des sucD Deletionsallels ausgewählt.

sucD-d1:
5´-CGA TGT GAT TGC GCT TGA TG -3´
sucD-d2:
5´-ACC TCA CGC ATA AGC TTC GCA TGC TCT GAA CCT TCC GAA C -
3´
sucD-d3:
5´-GTT CGG AAG GTT CAG AGC ATG CGA AGC TTA TGC GTG AGG T -
3´
sucD-d4:
5´-ATG AAG CCA GCG ACT GCA GA -3´

Die dargestellten Primer wurden von der Firma MWG Biotech (Ebersberg, Deutschland) synthetisiert und die PCR-Reaktion unter Verwendung der Pfu-Polymerase (Stratagene, Product. No. 600135, La Jolla, USA) und des PTC 100-Thermocyclers (MJ Research Inc., Waltham, USA) durchgeführt. Mit Hilfe der Polymerase-Kettenreaktion ermöglichen die Primer die Amplifikation eines sucD Allels mit interner Deletion. Das so amplifizierte Produkt wurde in einem 0,8%igen Agarosegel elektrophoretisch geprüft und ebenso wie bei Sanger et al. beschrieben (Proceedings of the National Academy of Sciences of the United States of America, 74:5463-5467, 1977) sequenziert.

### Beispiel 4

### 4.1 Integrationsmutagenese des sucC-Gens in dem Stamm DSM 5715

Der in Beispiel 3.1 genannte Vektor pCRBluntsucCint wurde nach der Elektroporationsmethode von Tauch et.al. (FEMS Microbiological Letters, 123:343-347 (1994)) in C. glutamicum DSM 5715 (EP 0 435 132) elektroporiert. Bei dem Stamm DSM 5715 handelt es sich um einen AEC resistenten L-Lysin-Produzenten. Der Vektor pCRBluntsucCint kann in DSM5715 nicht selbständig replizieren und bleibt nur dann in der Zelle erhalten, wenn er ins Chromosom von DSM 5715 integriert hat. Die Selektion von Klonen mit ins Chromosom integriertem pCRBluntsucCint erfolgte durch Ausplattieren des Elektroporationsansatzes auf LB Agar (Sambrook et al., Molecular Cloning: A Laboratory Manual, 2^{nd} Ed., Cold Spring Harbor Laboratory Press, Cold Spring Harbor, N.Y.), der mit 15 mg/l Kanamycin supplementiert worden war.

Für den Nachweis der Integration wurde das sucCint-Fragment nach der Methode "The DIG System Users Guide for Filter Hybridization" der Firma Boehringer Mannheim GmbH (Mannheim, Deutschland, 1993) mit dem Dig-Hybridisierungskit der Firma Boehringer markiert. Chromosomale DNA eines potentiellen Integranten wurde nach der Methode von Eikmanns et al. (Microbiology 140: 1817 - 1828 (1994)) isoliert und jeweils mit den Restriktionsenzymen SphI und HindIII geschnitten. Die entstehenden Fragmente wurden mittels der Agarosegel-Elektrophorese aufgetrennt und mit dem Dig-Hybrisierungskit der Firma Boehringer bei 68°C hybridisiert. Das in Beispiel 3.1 genannte Plasmid pCRBluntsucCint hatte innerhalb des chromosomalen sucC-Gens ins Chromosom von DSM5715 inseriert. Der Stamm wurde als DSM5715::pCRBluntsucCint bezeichnet.

### 4.2 Konstruktion des Austauschvektors pK18mobsacBsucDdel

Das in Beispiel 3.2 erhaltene sucD-Deletionsderivat wurde nach Auftrennung in einem Agarosegel (0,8%) mit Hilfe des Qiagenquick Gel Extraction Kit (Qiagen, Hilden, Germany) aus dem Agarosegel isoliert und mit dem mobilisierbaren Klonierungsvektor pK18mobsacB (Schäfer et al. (1994), Gene 14: 69-73) zur Ligation eingesetzt. Dieser wurde zuvor mit dem Restriktionsenzymen XmaI- und XbaI aufgespalten, mit dem sucD- Deletionsallel gemischt und mit T4-DNA-Ligase (Amersham- Pharmaecia, Freiburg, Germany) behandelt.

Anschließend wurde der E.coli Stamm DHSαmcr (Grant, 1990, Proceedings of the National Academy of Sciences U.S.A., 87:4645-4649) mit dem Ligationsansatz (Hanahan, In. DNA Cloning. A Practical Approach, Vol.1, ILR-Press, Cold Spring Habor, New York, 1989) elektroporiert. Die Selektion der Plasmid-tragenden Zellen erfolgte durch Ausplattieren des Tansformationsansatzes auf LB Agar(Sambrock et al., Molecular Cloning: A Laboratory Manual. 2^{nd} Ed. Cold Spring Habor, New York, 1989), der mit 25 mg/l Kanamycin supplementiert worden war.

Plasmid-DNA wurde aus einer Transformante mit Hilfe des QIAprep Spin Miniprep Kit der Firma Qiagen isoliert und das klonierte sucD-Deletionsallel mittels Sequenzierung durch die Firma MWG Biotech (Ebersberg, Deutschland) verifiziert. Das Plasmid wurde pK18mobsacBsucDdel genannt. Der Stamm wurde als E.coliDH5αmcr/pK18mobsacBsucDdel bezeichnet.

### 4.3 Deletionsmutagenese des sucD-Gens in dem C. glutamicum Stamm DSM 5715

Der in Beispiel 4.2 genannte Vektor pK18mobsacBsucDdel wurde nach der Elekroporationsmethode von Tauch et al., (1989 FEMS Microbiology Letters 123: 343-347) elekroporiert. Der Vektor kann in DSM 5715 nicht selbständig replizieren und bleibt nur dann in der Zelle erhalten, wenn er ins Chromosom integriert hat. Die Selektion von Klonen mit integriertem pK18mobsacBsucDdel erfolgte durch Ausplattieren des Elekroporationsansatzes auf LB-Agar (Sambrock et al., Molecular Cloning: A Laboratory Manual, 2^{nd} Ed., Cold Spring Habor, New York, 1989), der mit 15 mg/l Kanamycin supplementiert worden war. Angewachsene Klone wurden auf LB-Agarplatten mit 25 mg/l Kanamycin ausgestrichen und für 16 Stunden bei 33°C inkubiert.

Um die Excision des Plasmides zusammen mit der vollständigen chromosomalen Kopie des sucD-Gens zu erreichen, wurden die Klone anschließend auf LB-Agar mit 10% Sucrose angezogen. Das Plasmid pK18mobsacB enthält eine Kopie des sacB-Gens, welches Sucrose in die für C. glutamicum toxische Levansucrase umwandelt. Auf LB-Agar mit Sucrose wachsen daher nur solche Klone an, bei denen das integrierte pK18mobsacBsucDdel wiederum excisiert hat. Bei der Excision kann zusammen mit dem Plasmid entweder die vollständige chromosomale Kopie des sucD-Gens excisieren, oder die unvollständige Kopie mit der internen Deletion.

Um nachzuweisen, daß die unvollständige Kopie von sucD im Chromosom verblieben ist, wurde das Plasmid pK18mobsacBsucDdel Fragment nach der Methode "The DIG System Users Guide for Filter Hybridization" der Firma Boehringer Mannheim GmbH (Mannheim, Deutschland, 1993) mit dem Dig-Hybridisierungskit der Firma Boehringer markiert. Chromosomale DNA einer potentiellen Deletionsmutante wurde nach der Methode von Eikmanns et al. (Microbiology 140: 1817-1828 (1994)) isoliert und jeweils mit dem Restriktionsenzymen SphI und PstI in getrennten Ansätzen geschnitten. Die entstehenden Fragmente wurden mit Agarosegel-Elektrophorese aufgetrennt und mit dem Dig-Hybrisierungskit der Firma Boehringer bei 68°C hybridisiert. Anhand der enstandenen Fragmente konnte gezeigt werden, daß der Stamm DSM5715 seine vollständige Kopie des sucD-Gens verloren hat und stattdessen nur noch über die deletierte Kopie verfügt.
Der Stamm wurde als C. glutamicum DSM5715ΔsucD bezeichnet.

### Beispiel 5

### 5.1 Herstellung von L-Glutamat mit dem Stamm DSM 5715::pCRBluntsucCint

Der in Beispiel 4.1 erhaltene C. glutamicum Stamm DSM5715::pCRBluntsucCint wurde in einem zur Produktion von L-Glutamat geeigneten Nährmedium kultiviert und der Glutamatgehalt im Kulturüberstand bestimmt.

Dazu wurde der Stamm zunächst auf Agarplatte mit dem entsprechenden Antibiotikum (Hirn-Herz Agar mit Kanamycin (25 mg/l) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium Cg III verwendet.

| Medium Cg III | |
|---|---|
| NaCl | 2,5 g/l |
| Bacto-Pepton | 10 g/l |
| Bacto-Yeast-Extrakt | 10 g/l |
| Glucose (getrennt autoklaviert) | 2% (w/v) |
| Der pH-Wert wurde auf pH 7.4 eingestellt | |

Diesem wurde Kanamycin (25 mg/l) zugesetzt. Die Vorkultur wurde 16 Stunden bei 33°C bei 240 rpm auf dem Schüttler inkubiert. Von dieser Vorkultur wurde eine Hauptkultur angeimpft, so daß die Anfangs-OD (660 nm) der Hauptkultur 0,1 OD betrug. Für die Hauptkultur wurde das Medium MM verwendet

| Medium MM | |
|---|---|
| CSL (Corn Steep Liquor) | 5 g/l |
| MOPS (Morpholinopropansufonsäure) | 20 g/l |
| Glucose (getrennt autoklaviert) | 50g/l |

| Salze: | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO4 * 7 H₂O | 10 mg/l |
| MnSO₄ * H₂O | 5,0 mg/l |
| Biotin (sterilfiltriert) | 0,3 mg/l |
| Thiamin * HCl (sterilfiltriert) | 0,2 mg/l |
| Fumarat (sterilfiltriert) | 5,81 g/l |
| Leucin (sterilfiltriert) | 0,1 g/l |
| CaCO₃ | 25 g/l |

CSL, MOPS und die Salzlösung werden mit Ammoniakwasser auf pH 7 eingestellt und autoklaviert. Anschließend werden die sterilen Substrat- und Vitaminlösungen zugesetzt, sowie das trocken autoklavierte CaCO₃ zugesetzt.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchtigkeit.

Nach 24 Stunden wurde die OD bei einer Meßwellenlänge von 660 nm mit dem Biomek 1000 (Beckmann Instruments GmbH, München) ermittelt. Die gebildete Glutamatmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD (660 nm) | L-Glutamat mg/l |
|---|---|---|
| DSM5715 | 10,4 | 20 |
| DSM5715::pCRBlunt sucCint | 3,9 | 154 |

### 5.2 Herstellung von L-Glutamat mit dem Stamm DSM5715ΔsucD

Der in Beispiel 4.3 erhaltene C. glutamicum Stamm DSM5715/pK18mobsacBsucDdel wurde in einem zur Produktion von L-Glutamat geeigneten Nährmedium kultiviert und der Glutamatgehalt im Kulturüberstand bestimmt.

Dazu wurde der Stamm zunächst auf Agarplatte für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium CgIII verwendet. Diesem wurde Kanamycin (25 mg/l) zugesetzt. Die Vorkultur wurde 16 Stunden bei 33°C bei 240 rpm auf dem Schüttler inkubiert. Von dieser Vorkultur wurde eine Hauptkultur angeimpft, so daß die Anfangs-OD (660 nm) der Hauptkultur 0,1 OD betrug. Für die Hauptkultur wurde das Medium MM verwendet.

Die Kultivierung erfolgte in 10 ml Volumen in einem 100 ml Erlenmeyerkolben mit Schikanen. Die Kultivierung erfolgte bei 33°C und 80% Luftfeuchte.

Nach 72 Stunden wurde die OD bei einer Meßwellenlänge von 660 nm mit dem Biomek 1000 (Beckmann Instruments GmbH, München) ermittelt. Die gebildete Glutamatmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 2 ist das Ergebnis des Versuches dargestellt.

**Tabelle 2**

| Stamm | OD (660 nm) | L-Glutamat mg/l |
|---|---|---|
| DSM5715 | 8,1 | 7 |
| DSM5715ΔsucD | 13,3 | 33 |

### Folgende Figuren sind beigefügt:

### Figur 1: Karte des Plasmids pCRBluntsucCint.

Die verwendeten Abkürzungen und Bezeichnungen haben folgende Bedeutung.
- KmR:: Kanamycin Resistenz-Gen
- Zeocin:: Zeocin Resistenz-Gen
- HindIII: Schnittstelle des Restriktionsenzyms HindIII
- SphI: Schnittstelle des Restriktionsenzyms SphI
- EcoRI:: Schnittstelle des Restriktionsenzyms EcoRI
- sucCint:: internes Fragment des sucC-Gens
- ColE1 ori:: Replikationsursprung des Plasmides ColE1

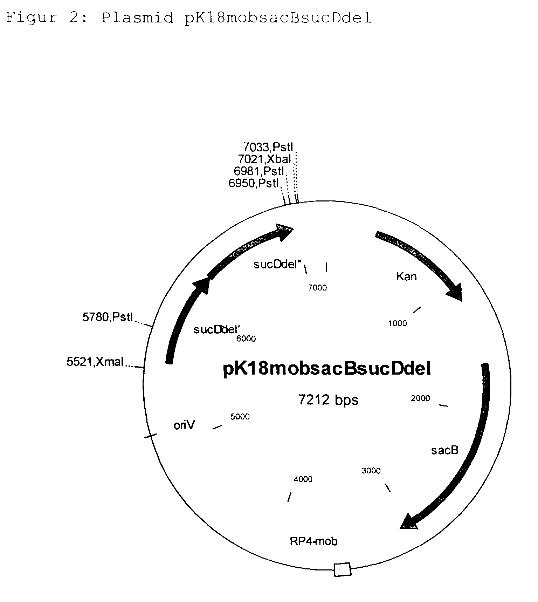

### Figur 2: Karte des Plasmids pK18mobsacBsucDdel

Die verwendeten Abkürzungen und Bezeichnungen haben folgende Bedeutung.
- oriV:: ColE1-ähnlicher Origin aus pMB1
- sacB: das für das Protein Levansucrose kodierende sacB-Gen
- RP4mob:: RP4-Mobilisierungs-Site
- Kan:: Resistenzgen für Kanamycin
- sucDdel:: deletiertes Allel des sucD-Gens aus C. glutamicum
- SphI:: Schnittstelle des Restriktionsenzyms SphI
- PstI:: Schnittstelle des Restriktionsenzyms PstI
- XmaI:: Schnittstelle des Restriktionsenzyms XmaI
- XbaI:: Schnittstelle für das Restriktionsnzym XbaI

## Patentansprüche

1. Isoliertes Polynukleotid, enthaltend eine für das sucC und/oder sucD-Gen kodierende Polynukleotidsequenz, ausgewählt aus der Gruppe
a) Polynukleotid, das mindestens zu 70 % identisch ist mit einem Polynukleotid, das für ein Polypeptid kodiert, das die Aminosäuresequenz von SEQ ID No. 2 enthält,
b) Polynukleotid, das mindestens zu 70 % identisch ist mit einem Polynukleotid, das für ein Polypeptid kodiert, das die Aminosäuresequenz von SEQ ID No. 3 enthält,
c) Polynukleotid, das für ein Polypeptid kodiert, das eine Aminosäuresequenz enthält, die zu mindestens 70% identisch ist mit der Aminosäuresequenz von SEQ ID No. 2,
d) Polynukleotid, das für ein Polypeptid kodiert, das eine Aminosäuresequenz enthält, die zu mindestens 70% identisch ist mit der Aminosäuresequenz von SEQ ID No. 3,
e) Polynukleotid, das komplementär ist zu den Polynukleotiden von a), b), c) oder d), und
f) Polynukleotid, enthaltend mindestens 15 aufeinanderfolgende Nukleotide der Polynukleotidsequenzen von a), b), c), d) oder e),
wobei das Polypeptid bevorzugt die Aktivität der Succinyl-CoA Synthetase aufweist.

2. Polynukleotide gemäß Anspruch 1, wobei das Polynukleotid eine in coryneformen Bakterien replizierbare, bevorzugt rekombinante DNA ist.

3. Polynukleotide gemäß Anspruch 1, wobei das Polynukleotid eine RNA ist.

4. Polynukleotide gemäß Anspruch 2, enthaltend die Nukleinsäuresequenz wie in SEQ ID NO 1 dargestellt.

5. Replizierbare DNA gemäß Anspruch 2 enthaltend
(i) die Nukleotidsequenz, gezeigt in SEQ ID NO 1, oder
(ii) mindestens eine Sequenz, die den Sequenzen (i) innerhalb des Bereichs der Degeneration des genetischen Codes entspricht, oder
(iii) mindestens eine Sequenz, die mit den zu den Sequenzen (i) oder (ii) komplementären Sequenzen hybridisiert, und gegebenenfalls
(iv) funktionsneutrale Sinnmutationen in (i).

6. Replizierbare DNA gemäß Anspruch 5, **dadurch gekennzeichnet**, daß die Hybridisierung unter einer Stringenz entsprechend höchstens 2x SSC durchgeführt wird.

7. Polynukleotidsequenz gemäß Anspruch 1, die für ein Polypeptid kodiert, das die in SEQ ID No. 2 dargestellte Aminosäuresequenz enthält.

8. Coryneforme Bakterien, in denen das sucC- und/oder sucD-Gen abgeschwächt wird.

9. Verfahren zur Herstellung von L-Aminosäuren,
insbesondere L-Lysin und/oder L-Glutamat,
**dadurch gekennzeichnet**,
daß man folgende Schritte durchführt,
a) Fermentation der die gewünschte L-Aminosäure produzierenden Bakterien, in denen man zumindest das sucC- und/oder sucD-Gen oder dafür kodierende Nukleotidsequenzen abschwächt, insbesondere ausschaltet;
b) Anreicherung der L- Aminosäure im Medium oder in den Zellen der Bakterien, und
c) Isolieren der L-Aminosäure.

10. Verfahren gemäß Anspruch 9, **dadurch gekennzeichnet**, daß man Bakterien einsetzt, in denen man zusätzlich weitere Gene des Biosyntheseweges der gewünschten L-Aminosäure verstärkt.

11. Verfahren gemäß Anspruch 9, **dadurch gekennzeichnet**, daß man Bakterien einsetzt, in denen die Stoffwechselwege zumindest teilweise ausgeschaltet sind, die die Bildung der gewünschten L-Aminosäure verringern.

12. Verfahren gemäß Anspruch 9, **dadurch gekennzeichnet**, daß man die Expression des (der) Polynukleotids(e), das(die) für das sucC- bzw. sucD-Gen kodiert (kodieren), abschwächt, insbesondere ausschaltet.

13. Verfahren gemäß Anspruch 9, **dadurch gekennzeichnet**, daß man die katalytischen Eigenschaften des Polypeptids (Enzymproteins) herabsetzt, für das die Polynukleotide sucC und sucD kodieren.

14. Verfahren gemäß Anspruch 9, **dadurch gekennzeichnet**, daß man für die Herstellung von L-Aminosäuren, Mikroorganismen fermentiert, in denen man gleichzeitig eines oder mehrere der Gene, ausgewählt aus der Gruppe
14.1 das für die Dihydrodipicolinat-Synthase kodierende dapA-Gen,
14.2 das für die Pyruvat-Carboxylase kodierende pyc-Gen,
14.3 das für die Glyceraldehyd-3-Phosphat Dehydrogenase kodierende gap-Gen,
14.4 das für die Triosephosphat Isomerase kodierende Gen tpi,
14.5 das für die 3-Phosphoglycerat Kinase kodierende Gen pgk,
14.6 das für die Malat:Chinon Oxidoreduktase kodierende mqo-Gen,
14.7 das für den L-Lysin-Export kodierende lysE-Gen,
14.8 das für eine feed-back resistente Aspartatkinase kodierende Gen lysC,
14.9 das für das Zwa1-Protein kodierende Gen zwa1
verstärkt bzw. überexprimiert.

15. Verfahren gemäß Anspruch 9, **dadurch gekennzeichnet**, daß man zur Herstellung von L-Aminosäuren coryneforme Mikroorganismen fermentiert, in denen man gleichzeitig eines oder mehrere der Gene, ausgewählt aus der Gruppe
15.1 das für die Phosphoenolpyruvat-Carboxykinase kodierende Gen pck,
15.2 das für die Glucose-6-Phosphat Isomerase kodierende Gen pgi,
15.3 das für die Pyruvat-Oxidase kodierende Gen poxB
15.4 das für das Zwa2-Protein kodierende Gen zwa2
abschwächt.

16. Coryneforme Bakterien, die einen Vektor enthalten, der Teile des Polynukleotids gemäß Anspruch 1, mindestens aber 15 aufeinanderfolgende Nukleotide der beanspruchten Sequenz, trägt.

17. Verfahren gemäß einem oder mehreren der vorhergehenden Ansprüche, **dadurch gekennzeichnet**, daß man Mikroorganismen der Art Corynebacterium glutamicum einsetzt.

18. Verfahren zum Auffinden von RNA, cDNA und DNA, um Nukleinsäuren, beziehungsweise Polynukleotide oder Gene zu isolieren, die für Succinyl-CoA Synthase kodieren oder eine hohe Ähnlichkeit der Sequenz des sucC und/oder sucD-Gens aufweisen, **dadurch gekennzeichnet**, daß man das Polynukleotid, enthaltend die Polynukleotidsequenzen gemäß den Ansprüchen 1, 2, 3 oder 4, als Hybridisierungssonden einsetzt.


SEQUENZPROTOKOLL

<110> Degussa-Hüls AG

<120> Neue für die Gene sucC und sucD kodierende
      Nukleotidsequenzen

<130> 990171 BT

<140>
<141>

<160> 3

<170> PatentIn Ver. 2.1

<210> 1
<211> 2410
<212> DNA
<213> Corynebacterium glutamicum

<220>
<221> CDS
<222> (142)..(1347)
<223> sucC

<220>
<221> CDS
<222> (1372)..(2253)
<223> sucD

<400> 1
gcaccacgga tccaattttg ttgcaatttg caaagtttac agtgttagac ttcacaatac 60

gatcatattg gtgagttgaa acacttactt ttacgggaag actttgttaa agacgcagaa 120

ggctctaagc atgggccgga a atg gaa ttg gca gtg gat ctt ttt gaa tac 171
                        Met Glu Leu Ala Val Asp Leu Phe Glu Tyr
                          1               5                  10

caa gca cgg gac ctc ttt gaa acc cat ggt gtg cca gtg ttg aag gga 219
Gln Ala Arg Asp Leu Phe Glu Thr His Gly Val Pro Val Leu Lys Gly
                 15                  20                  25

att gtg gca tca aca cca gag gcg gcg agg aaa gcg gct gag gaa atc 267
Ile Val Ala Ser Thr Pro Glu Ala Ala Arg Lys Ala Ala Glu Glu Ile
             30                  35                  40

ggc gga ctg acc gtc gtc aag gct cag gtc aag gtg ggc gga cgt ggc 315
Gly Gly Leu Thr Val Val Lys Ala Gln Val Lys Val Gly Gly Arg Gly
         45                  50                  55

aag gcg ggt ggc gtc cgt gtg gca ccg acg tcg gct cag gct ttt gat 363
Lys Ala Gly Gly Val Arg Val Ala Pro Thr Ser Ala Gln Ala Phe Asp
     60                  65                  70

gct gcg gat gcg att ctc ggc atg gat atc aaa gga cac act gtt aat 411
Ala Ala Asp Ala Ile Leu Gly Met Asp Ile Lys Gly His Thr Val Asn
75                  80                  85                  90

cag gtg atg gtg gcg cag ggc gct gac att gct gag gaa tac tat ttc 459
Gln Val Met Val Ala Gln Gly Ala Asp Ile Ala Glu Glu Tyr Tyr Phe
                95                  100                 105

tcc att ttg ttg gat cgc gcg aat cgt tcg tat ctg gct atg tgc tct 507
Ser Ile Leu Leu Asp Arg Ala Asn Arg Ser Tyr Leu Ala Met Cys Ser
            110                 115                 120

gtt gaa ggt ggc atg gag atc gag atc ctg gcg aag gaa aag cct gaa 555
Val Glu Gly Gly Met Glu Ile Glu Ile Leu Ala Lys Glu Lys Pro Glu
        125                 130                 135

gct ttg gca aag gtg gaa gtg gat ccc ctc act ggt att gat gag gac 603
Ala Leu Ala Lys Val Glu Val Asp Pro Leu Thr Gly Ile Asp Glu Asp
    140                 145                 150

aaa gcg cgg gag att gtc act gct gct ggc ttt gaa act gag gtg gca 651
Lys Ala Arg Glu Ile Val Thr Ala Ala Gly Phe Glu Thr Glu Val Ala
155                 160                 165                 170

gag aaa gtc att ccg gtg ctg atc aag atc tgg cag gtg tat tac gaa 699
Glu Lys Val Ile Pro Val Leu Ile Lys Ile Trp Gln Val Tyr Tyr Glu
                175                 180                 185

gag gaa gca aca ctc gtt gag gtg aac ccg ttg gtg ctc acg gat gac 747
Glu Glu Ala Thr Leu Val Glu Val Asn Pro Leu Val Leu Thr Asp Asp
            190                 195                 200

ggc gat gtg att gcg ctt gat ggc aag atc acg ctg gat gat aac gct 795
Gly Asp Val Ile Ala Leu Asp Gly Lys Ile Thr Leu Asp Asp Asn Ala
        205                 210                 215

gat ttc cgc cat gat aac cgt ggt gcg ttg gct gaa tct gcc ggt ggc 843
Asp Phe Arg His Asp Asn Arg Gly Ala Leu Ala Glu Ser Ala Gly Gly
    220                 225                 230

ttg gac att ttg gaa ctg aag gcc aag aag aat gat ctg aac tac gtg 891
Leu Asp Ile Leu Glu Leu Lys Ala Lys Lys Asn Asp Leu Asn Tyr Val
235                 240                 245                 250

aaa ctt gat ggc tct gtg ggc atc att ggc aat ggt gca ggt ttg gtg 939
Lys Leu Asp Gly Ser Val Gly Ile Ile Gly Asn Gly Ala Gly Leu Val
                255                 260                 265

atg tcc acg ttg gat atc gtg gct gca gct ggt gaa cgc cat ggt ggg 987
Met Ser Thr Leu Asp Ile Val Ala Ala Ala Gly Glu Arg His Gly Gly
            270                 275                 280

cag cgc ccc gcg aac ttc cta gac att ggt ggc gga gca tca gct gaa 1035
Gln Arg Pro Ala Asn Phe Leu Asp Ile Gly Gly Gly Ala Ser Ala Glu
        285                 290                 295

tcg atg gct gct ggt ctc gat gtg atc ctt ggg gat agc cag gta cgc 1083
Ser Met Ala Ala Gly Leu Asp Val Ile Leu Gly Asp Ser Gln Val Arg
    300                 305                 310

agt gtg ttt gtg aat gtg ttt ggt ggc atc acc gcg tgt gat gtg gtg 1131
Ser Val Phe Val Asn Val Phe Gly Gly Ile Thr Ala Cys Asp Val Val
315                 320                 325                 330

gca aag gga atc gtt gga gct ttg gat gtg ctc ggc gat caa gca acg 1179
Ala Lys Gly Ile Val Gly Ala Leu Asp Val Leu Gly Asp Gln Ala Thr
                335                 340                 345

aag cct ctt gtg gtg cgc ctt gat ggc aac aac gtg gtg gaa ggc aga 1227
Lys Pro Leu Val Val Arg Leu Asp Gly Asn Asn Val Val Glu Gly Arg
            350                 355                 360

cga atc ctc gcg gaa tat aac cac cct ttg gtc acc gtt gtg gag ggt 1275
Arg Ile Leu Ala Glu Tyr Asn His Pro Leu Val Thr Val Val Glu Gly
        365                 370                 375

atg gat gca gcg gct gat cac gct gcc cat ttg gcc aat ctt gcc cag 1323
Met Asp Ala Ala Ala Asp His Ala Ala His Leu Ala Asn Leu Ala Gln
    380                 385                 390

cac ggc cag ttc gca acc gct aat tagttaagga gcacctgttt aatc atg 1374
His Gly Gln Phe Ala Thr Ala Asn Met
395                 400

tct att ttt ctc aat tca gat tcc cgc atc atc att cag ggc att acc 1422
Ser Ile Phe Leu Asn Ser Asp Ser Arg Ile Ile Ile Gln Gly Ile Thr
    405                 410                 415

ggt tcg gaa ggt tca gag cat gcg cgt cga att tta gcc tct ggt gcg 1470
Gly Ser Glu Gly Ser Glu His Ala Arg Arg Ile Leu Ala Ser Gly Ala
420                 425                 430                 435

aag ctc gtg ggt ggc acc aac ccc cgc aaa gct ggg caa acc att ttg 1518
Lys Leu Val Gly Gly Thr Asn Pro Arg Lys Ala Gly Gln Thr Ile Leu
                440                 445                 450

atc aat gac act gag ttg cct gta ttt ggc act gtt aag gaa gca atg 1566
Ile Asn Asp Thr Glu Leu Pro Val Phe Gly Thr Val Lys Glu Ala Met
            455                 460                 465

gag gaa acg ggt gcg gat gtc acc gta att ttc gtt cct cca gcc ttt 1614
Glu Glu Thr Gly Ala Asp Val Thr Val Ile Phe Val Pro Pro Ala Phe
        470                 475                 480

gcc aaa gct gcg atc att gaa gct atc gac gct cac atc cca ctg tgc 1662
Ala Lys Ala Ala Ile Ile Glu Ala Ile Asp Ala His Ile Pro Leu Cys
    485                 490                 495

gtg att att act gag ggc atc cca gtg cgt gac gct tct gag gcg tgg 1710
Val Ile Ile Thr Glu Gly Ile Pro Val Arg Asp Ala Ser Glu Ala Trp
500                 505                 510                 515

gct tat gcc aag aag gtg gga cac acc cgc atc att ggc cct aac tgc 1758
Ala Tyr Ala Lys Lys Val Gly His Thr Arg Ile Ile Gly Pro Asn Cys
                520                 525                 530

cca ggc att att act ccc ggc gaa tct ctt gcg gga att acg ccg gca 1806
Pro Gly Ile Ile Thr Pro Gly Glu Ser Leu Ala Gly Ile Thr Pro Ala
            535                 540                 545

aac att gca ggt tcc ggc ccg atc ggg ttg atc tca aag tcg gga aca 1854
Asn Ile Ala Gly Ser Gly Pro Ile Gly Leu Ile Ser Lys Ser Gly Thr
        550                 555                 560

ctg act tat cag atg atg tac gaa ctt tca gat att ggc att tct acg 1902
Leu Thr Tyr Gln Met Met Tyr Glu Leu Ser Asp Ile Gly Ile Ser Thr
    565                 570                 575

gcg att ggt att ggc ggt gac cca atc atc ggt aca acc cat atc gac 1950
Ala Ile Gly Ile Gly Gly Asp Pro Ile Ile Gly Thr Thr His Ile Asp
580                 585                 590                 595

gct ctg gag gcc ttt gaa gct gat cct gag acc aag gca atc gtc atg 1998
Ala Leu Glu Ala Phe Glu Ala Asp Pro Glu Thr Lys Ala Ile Val Met
                600                 605                 610

atc ggt gag atc ggt gga gat gca gag gaa cgc gct gct gac ttc att 2046
Ile Gly Glu Ile Gly Gly Asp Ala Glu Glu Arg Ala Ala Asp Phe Ile
            615                 620                 625

tct aag cac gtg aca aaa cca gtt gtg ggt tac gtg gca ggc ttt acc 2094
Ser Lys His Val Thr Lys Pro Val Val Gly Tyr Val Ala Gly Phe Thr
        630                 635                 640

gcc cct gaa gga aag acc atg ggg cat gct ggc gcc atc gtg aca ggt 2142
Ala Pro Glu Gly Lys Thr Met Gly His Ala Gly Ala Ile Val Thr Gly
    645                 650                 655

tca gaa ggc act gcg cga gca aag aag cat gca ttg gag gcc gtg ggt 2190
Ser Glu Gly Thr Ala Arg Ala Lys Lys His Ala Leu Glu Ala Val Gly
660                 665                 670                 675

gtt cgc gtg gga aca act ccg agt gaa acc gcg aag ctt atg cgt gag 2238
Val Arg Val Gly Thr Thr Pro Ser Glu Thr Ala Lys Leu Met Arg Glu
                680                 685                 690

gta gtt gca gct ttg taactaacag gccacagatc ttagctttga ccagcggatt 2293
Val Val Ala Ala Leu
            695

tgtggctaat cgcccggtct gtgtagagta ttcatctgtg cgcaggacag tgtgacaaac 2353

actgaatagt gcatggcttt aaggccctgt ggcgcagttg gttagcgcgc cgccctg 2410

<210> 2
<211> 402
<212> PRT
<213> Corynebacterium glutamicum

<400> 2
Met Glu Leu Ala Val Asp Leu Phe Glu Tyr Gln Ala Arg Asp Leu Phe
1               5                   10                  15

Glu Thr His Gly Val Pro Val Leu Lys Gly Ile Val Ala Ser Thr Pro
            20                  25                  30

Glu Ala Ala Arg Lys Ala Ala Glu Glu Ile Gly Gly Leu Thr Val Val
        35                  40                  45

Lys Ala Gln Val Lys Val Gly Gly Arg Gly Lys Ala Gly Gly Val Arg
    50                  55                  60

Val Ala Pro Thr Ser Ala Gln Ala Phe Asp Ala Ala Asp Ala Ile Leu
65                  70                  75                  80

Gly Met Asp Ile Lys Gly His Thr Val Asn Gln Val Met Val Ala Gln
                85                  90                  95

Gly Ala Asp Ile Ala Glu Glu Tyr Tyr Phe Ser Ile Leu Leu Asp Arg
            100                 105                 110

Ala Asn Arg Ser Tyr Leu Ala Met Cys Ser Val Glu Gly Gly Met Glu
        115                 120                 125

Ile Glu Ile Leu Ala Lys Glu Lys Pro Glu Ala Leu Ala Lys Val Glu
    130                 135                 140

Val Asp Pro Leu Thr Gly Ile Asp Glu Asp Lys Ala Arg Glu Ile Val
145                 150                 155                 160

Thr Ala Ala Gly Phe Glu Thr Glu Val Ala Glu Lys Val Ile Pro Val
                165                 170                 175

Leu Ile Lys Ile Trp Gln Val Tyr Tyr Glu Glu Glu Ala Thr Leu Val
            180                 185                 190

Glu Val Asn Pro Leu Val Leu Thr Asp Asp Gly Asp Val Ile Ala Leu
        195                 200                 205

Asp Gly Lys Ile Thr Leu Asp Asp Asn Ala Asp Phe Arg His Asp Asn
    210                 215                 220

Arg Gly Ala Leu Ala Glu Ser Ala Gly Gly Leu Asp Ile Leu Glu Leu
225                 230                 235                 240

Lys Ala Lys Lys Asn Asp Leu Asn Tyr Val Lys Leu Asp Gly Ser Val
                245                 250                 255

Gly Ile Ile Gly Asn Gly Ala Gly Leu Val Met Ser Thr Leu Asp Ile
            260                 265                 270

Val Ala Ala Ala Gly Glu Arg His Gly Gly Gln Arg Pro Ala Asn Phe
        275                 280                 285

Leu Asp Ile Gly Gly Gly Ala Ser Ala Glu Ser Met Ala Ala Gly Leu
    290                 295                 300

Asp Val Ile Leu Gly Asp Ser Gln Val Arg Ser Val Phe Val Asn Val
305                 310                 315                 320

Phe Gly Gly Ile Thr Ala Cys Asp Val Val Ala Lys Gly Ile Val Gly
                325                 330                 335

Ala Leu Asp Val Leu Gly Asp Gln Ala Thr Lys Pro Leu Val Val Arg
            340                 345                 350

Leu Asp Gly Asn Asn Val Val Glu Gly Arg Arg Ile Leu Ala Glu Tyr
        355                 360                 365

Asn His Pro Leu Val Thr Val Val Glu Gly Met Asp Ala Ala Ala Asp
    370                 375                 380

His Ala Ala His Leu Ala Asn Leu Ala Gln His Gly Gln Phe Ala Thr
385                 390                 395                 400

Ala Asn


<210> 3
<211> 294
<212> PRT
<213> Corynebacterium glutamicum

<400> 3
Met Ser Ile Phe Leu Asn Ser Asp Ser Arg Ile Ile Ile Gln Gly Ile
  1               5                  10                  15

Thr Gly Ser Glu Gly Ser Glu His Ala Arg Arg Ile Leu Ala Ser Gly
             20                  25                  30

Ala Lys Leu Val Gly Gly Thr Asn Pro Arg Lys Ala Gly Gln Thr Ile
         35                  40                  45

Leu Ile Asn Asp Thr Glu Leu Pro Val Phe Gly Thr Val Lys Glu Ala
     50                  55                  60

Met Glu Glu Thr Gly Ala Asp Val Thr Val Ile Phe Val Pro Pro Ala
 65                  70                  75                  80

Phe Ala Lys Ala Ala Ile Ile Glu Ala Ile Asp Ala His Ile Pro Leu
                 85                  90                  95

Cys Val Ile Ile Thr Glu Gly Ile Pro Val Arg Asp Ala Ser Glu Ala
            100                 105                 110

Trp Ala Tyr Ala Lys Lys Val Gly His Thr Arg Ile Ile Gly Pro Asn
        115                 120                 125

Cys Pro Gly Ile Ile Thr Pro Gly Glu Ser Leu Ala Gly Ile Thr Pro
    130                 135                 140

Ala Asn Ile Ala Gly Ser Gly Pro Ile Gly Leu Ile Ser Lys Ser Gly
145                 150                 155                 160

Thr Leu Thr Tyr Gln Met Met Tyr Glu Leu Ser Asp Ile Gly Ile Ser
                165                 170                 175

Thr Ala Ile Gly Ile Gly Gly Asp Pro Ile Ile Gly Thr Thr His Ile
            180                 185                 190

Asp Ala Leu Glu Ala Phe Glu Ala Asp Pro Glu Thr Lys Ala Ile Val
        195                 200                 205

Met Ile Gly Glu Ile Gly Gly Asp Ala Glu Glu Arg Ala Ala Asp Phe
    210                 215                 220

Ile Ser Lys His Val Thr Lys Pro Val Val Gly Tyr Val Ala Gly Phe
225                 230                 235                 240

Thr Ala Pro Glu Gly Lys Thr Met Gly His Ala Gly Ala Ile Val Thr
                245                 250                 255

Gly Ser Glu Gly Thr Ala Arg Ala Lys Lys His Ala Leu Glu Ala Val
            260                 265                 270

Gly Val Arg Val Gly Thr Thr Pro Ser Glu Thr Ala Lys Leu Met Arg
        275                 280                 285

Glu Val Val Ala Ala Leu
    290